(19) Europäisches Patentamt
European Patent Office
Office européen des brevets

(11) Veröffentlichungsnummer: **0 129 773**
**B1**

# (12) EUROPÄISCHE PATENTSCHRIFT

(45) Veröffentlichungstag der Patentschrift: **12.09.90**

(21) Anmeldenummer: **84106726.7**

(22) Anmeldetag: **13.06.84**

(51) Int. Cl.[5]: **C 07 D 231/36,**
C 07 D 491/10, A 01 N 47/18,
A 01 N 47/24 // (C07D491/10,
311:00, 231:00)

## (54) Insektizide Carbaminsäureester.

(30) Priorität: **15.06.83 DE 3321519**
**28.09.83 DE 3335010**

(43) Veröffentlichungstag der Anmeldung:
**02.01.85 Patentblatt 85/01**

(45) Bekanntmachung des Hinweises auf die Patenterteilung:
**12.09.90 Patentblatt 90/37**

(84) Benannte Vertragsstaaten:
**BE CH DE FR GB IT LI NL**

(56) Entgegenhaltungen:
**DE-A-2 630 912**
**US-A-3 149 115**

(73) Patentinhaber: **BASF Aktiengesellschaft**
**Carl-Bosch-Strasse 38**
**D-6700 Ludwigshafen (DE)**

(72) Erfinder: **Lange, Arno, Dr.**
**Oberes Geistal 3b**
**D-6702 Bad Duerkheim (DE)**
Erfinder: **Merger, Franz, Dr.**
**Max-Slevogt-Strasse 25**
**D-6710 Frankenthal (DE)**
Erfinder: **Hettinger, Peter, Dr.**
**Schloss-Strasse 3**
**D-6803 Edingen-Neckarhausen (DE)**
Erfinder: **Adolphi, Heinrich, Dr.**
**Kalmitweg 11**
**D-6703 Limburgerhof (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Gegenstand der Erfindung sind neue Carbaminsäureester (Carbamate) der Formel I

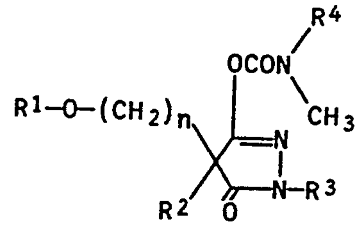

(I),

in der entweder $R^1$ einen verzweigten oder unverzweigten Alkylrest mit bis zu 4 C-Atomen und $R^2$ einen verzweigten oder unverzweigten Alkylrest mit bis zu 6 Kohlenstoffatomen bedeutet, oder $R^1$ und $R^2$ zusammen mit dem Molekülteil, das sie einschließen, einen Tetrahydrofuran- oder Tetrahydropyranring bilden, n die Zahl 1 oder 2 bedeutet, $R^3$ einen verzweigten oder unverzweigten Alkylrest mit bis zu 6 C-Atomen oder einen Cycloalkylrest mit 3—6 C-Atomen und $R^4$ den Methyl- oder Methoxyrest bedeuten sowie Verfahren zu ihrer Hestellung und Schädlingsbekämpfungsmittel, die diese Carbamate als Wirkstoff enthalten.

Aus der DE—A 26 30 912 ist bekannt, daß den Verbindungen I strukturell ähnliche Carbamate, z.B. mit einer $C_1$—$C_4$-Alkylgrupe anstelle der beanspruchten Alkoxyalkylgruppe in 4-Position eine gute, jedoch nicht immer ausreichende insektizide Wirksamkeit aufweisen.

Die neuen Carbamate sind, je nach Substitution, gelbliche bis farblose Öle oder farblose feste Stoffe mit starker biologischer Wirkung, die ihre Verwendung als Insektizide oder Acarizide zur Bekämpfung tierischer Schädlinge erlaubt. Sie zeichnen sich insbesondere durch eine gute Wirkung gegen saugende Insekten aus.

Der Rest $R^1$ kann z.B. der Methyl-, Ethyl, Isopropyl- oder Isobutylrest sein.

Verzweigte oder unverzweigte Alkylreste für das Symbol $R^2$ in Formel I sind Methyl, Ethyl, Propyl, Isopropyl sowie die Butylreste. Verzweigte oder unverzweigte Alkylreste für $R^3$ sind Methyl, Ethyl, Propyl, Isopropyl, die Butyl-, Pentyl und Hexylreste.

Man kann die neuen Carbamate erhalten, indem man ein entsprechendes Salz eines Pyrazolinons (II) mit einem entsprechenden Carbaminsäurehalogenid umsetzt:

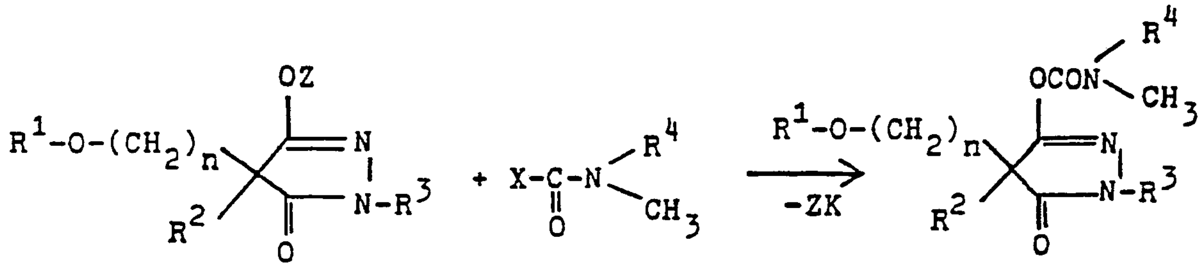


II III I

Dabei steht Z für ein einwertiges Kation, vorzugsweise eines Alkalimetalls oder ein Äquivalent eines mehrwertigen Kations und X für Halogen, vorzugsweise Chlor oder Brom.

Die Umsetzung läßt sich bequem in einem Lösungs- oder Verdünnungsmittel durchführen. Hierfür sind beispielsweise geeignet: Ether, wie Diethylether, Diisopropylether, Tetrahydrofuran, Dioxan, Diglykoldimethylether; Ketone, wie Aceton, Methylethylketon, Diethylketon, Diisopropylketon; chlorierte alphatische Kohlenwasserstoffe, wie Dichlormethan, Trichlormethan, Tetrachlorkohlenstoff, 1,1-Dichlorethan, 1,2-Dichlorethan; aromatishe Kohlenwasserstoffe, wie Toluol, Xylole und Chlorbenzole; Dimethylformamid; Nitromethan; Nitrile, wie Acetonitril, Propionitril.

Zur Durchführung des Verfahrens setzt man die Ausgangsstoffe meist in stöchiometrischem Verhältnis ein. Ein Überschuß von bis zu 200% des — im allgemeinen wohlfeileren — Carbamoylhalogenids begünstigt den Reaktionsablauf.

Die Reaktionstemperatur kann innerhalb eines größeren Bereiches variiert werden. Im allgemeinen arbeitet man bei einer Temperatur von bis zu 100°C, vorzugsweise bei 30 bis 90°C; der Siedepunkt des Verdünnungsmittels kann die Temperatur nach oben begrenzen. Als Katalysator kann ein Kronenether zugesetzt werden.

Die als Ausgangsverbindungen verwendeten Salze der (Hydroxy)pyrazolinone — diese Salze können auch als Salze von Pyrazolidindione bezeichnet werden — können nach bekannten Methoden (Helv. Chim. Acta. *36*, 74 ff (1953)) durch Umsetzung von entsprechenden disubstituierten Malonsäuredialkylestern (IV)

mit monosubstituierten Hydrazinen (V) in Gegenwart geeigneter Kondensationsmittel, beispielsweise Natriummethylat, hergestellt werden:

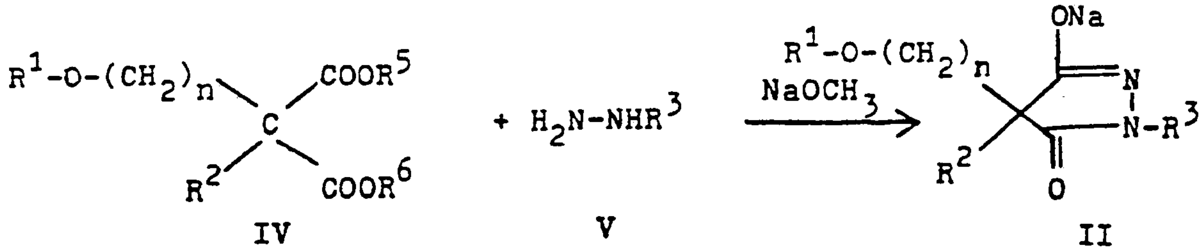


IV V II

$R^5$ und $R^6$ bedeuten vorzugsweise Methyl oder Ethyl.

Nach diesem Verfahren lassen sich die Salze der Pyrazolinone in guter Ausbeute und Reinheit herstellen. Die Salze werden z.B. isoliert, indem man nach beendeter Methylatzugabe das entstandene Methanol zusammen mit einem Lösungsmittel (z.B. Toluol, Xylol, Cumol, Chlorbenzol, Cyclohexan) abdestilliert, bis reines Lösungsmittel übergeht. Das anfallende kristalline Salz wird vom Lösungsmittel abgetrennt und getrocknet. Das Salz, insbesondere das Natriumsalz, kann ohne weitere Reinigung verwendet werden.

Der für die Herstellung der Pyrazolinon-Salze benötigte Malonester IV kann nach bekannten Methoden aus der freien Säure dagestellt werden. (Weygand-Hilgetag, Org. Chem. Experimentierkunst 1970, 4. Auflage, Seite 380—384.) Die freien Säuren kann man beispielsweise nach einem in Ar. 297 (4), S. 219 ff (1964) angegebenen Verfahren aus 2-Alkylmalonsäuren und Chlormethyl-alkylethern erhalten.

Allerdings ist die Herstellung bestimmter freier 2-Alkylmalonsäuren aus Chlormethylethern nicht der günstigste Weg, vor allem wegen der bekannten Gefährlichkeit der als kanzerogen geltenden Chlomethylether.

Es wird daher vorgeschlagen, die besonders wichtigen Malonester IV, bei denen n = 1 ist, ausgehend von 2-Alkylacroleinen V, Formaldehyd und n-Alkanolen, herzustellen, aus denen in Gegenwart eines tertiären Amins zunächst 2-Hydroxymethyl-aldehyde VI gebildet werden, die man, beispielsweise mit Salpetersäure, zu entsprechenden Malonsäuren VII oxidieren und dann verestern kann.

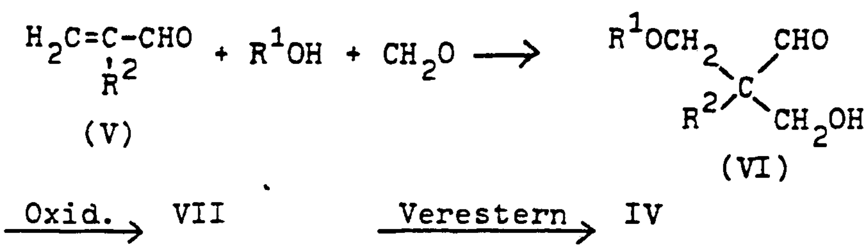


(V) (VI) VII IV

Die Umsetzung von V zu VI verläuft unterhalb von 150°C (z.B. bei 60 bis 100°C) vorzugsweise in wäßriger Umgebung (d.h. mit wäßrigem Formaldehyd) bei atmosphärischem Druck, wobei ein leichter Überschuß an Formaldehyd und ein höherer Überschuß an Alkohol (z.B. 4 bis 6 mol je mol Acrolein) zweckmäßig ist. Oxidation und Veresterung sind bekannte Verfahren, die für ähnliche Stoffe beschrieben sind.

Herstellbeispiel für Ethylmethoxymethylmalonsäuredimethylester

a) Herstellung von 2-Methoximethyl-2-hydroximethyl-butanal

Zu einer Mischung von 1600 ml Methanol, 500 g 30%iger Formaldehydlösung und 328 g 2-Ethylacrolein fügt man innerhalb 10 min 41 g Triethylamin zu und hält die Lösung dann 7 Stunden lang auf 78°C. Nach dem Abdestillieren niedersiedender Komponenten werden durch Vakuumdestillation 390 g der Titelverbindung (67% bezogen auf 2-Ethylacrolein) mit einem Siedepunkt bei 3 mbar von 95 bis 100°C isoliert.

b) Herstellung von Methoximethylethylmalonsäure

Zu 400 ml 66%iger Salpetersäure ($d^{20}$ = 1,40) fügt man zunächst 100 mg $MnCl_2 \cdot H_2O$ und dann bei 40°C innerhalb von 45 Minuten 140 g 2-Methoximethyl-2-hydroximethylbutanal. Man kühlt auf 0°C ab, filtriert die ausgefallene Säure ab und trocknet.

Ausbeute: 105 g entspr. 62% bezogen auf das Aldol

Schmelzpunkt: 72 bis 75°C unter Decarboxilierung.

c) Herstellung des Malonesters

In 500 ml Aceton werden 225 g wasserfreie Pottasche und 144 g Ethylmethoxymethylmalonsäure vorgelegt. Man tropft 206 g Dimethylsulfat zu und rührt 5 Stunden am Rückfluß. Dann wird kalt abgesaugt

und das eingeengte Filtrat in Chloroform aufgenommen. Man wäscht mit Wasser und wäßriger Natriumbicarbonatlösung und trocknet. Es wird eingeengt und destilliert.

141,3 g vom $Kp_{0,1}$: 48 bis 57°C.

Herstellbeispiel für Tetrahydropyran-3,3-dicarbonsäuremethylester

In 74 g Methanol werden 57,5 g Tetrahydropyran-3,3-dicarbonsäure vorgelegt und bei 10 bis 20°C HCl-Gas bis zur Sättigung eingeleitet. Man rührt 4 h bei Raumtemperatur nach und setzt 130 ml Toluol zu. Die Toluolphase wird abgetrennt, getrocknet, eingeengt und destilliert.

41,6 g vom $Kp_{0,01}$: 85 bis 90°C.

Beispiel 1

2-Methyl, 4-ethyl, 4-methoxymethyl, 5-dimethylcarbamoyloxypyrazolinon-(3)

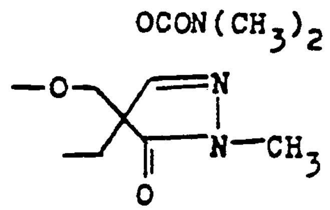

348 g Ethyl-methoxymethylmalonsäuredimethylester werden in 1,7 l Xylol gelöst. Dann werden 87 g Methylhydrazin und 307 g 30 %ige Natriummethylat-Lösung zugetropft. Man destilliert nun das Methanol unter gleichzeitiger Xylolzugabe ab, bis reines Xylol übergeht. Das Natriumsalz des Pyrazolidons wird abgesaugt, mit Ether gewaschen und getrocknet: 317 g. 208 g davon werden in 800 ml Aceton vorgelegt. Man setzt 0,1 g 15-Krone-5 zu und tropft unter Rückfluß 130 g Dimethylcarbamoylchlorid zu. Man hält weitere 3 Stunden am Rückfluß, saugt kalt ab und engt ein. Das Kristallisat wird scharf abgesaugt und mit Hexan gewaschen. Ausbeute 147,1 g; vom Schmelzpunkt 80 bis 83°C.

Elementaranalyse

| | C | H | N |
|---|---|---|---|
| Ber.: | 51,35% | 7,44% | 16,33% |
| Gef.: | 51,2% | 7,5% | 16,4% |

Beispiel 2

7-Oxa-2,3-diaza, 2-methyl, spiro[4,5]dec-3-en-1-on-4-yl-N,N-dimethylcarbaminsäureester

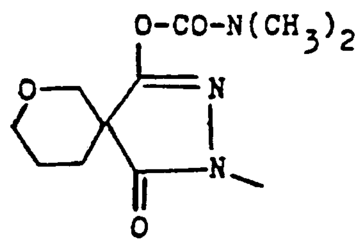

a) Herstellung von 3,3-Biscarboxi-tetrahydropyran

Man gibt zu einer Mischung von 600 g Salpetersäure ($d^{20}$ = 1,40) und 0,1 g $MnCl_2 \cdot H_2O$ bei 30 bis 35°C innerhalb 90 Minuten 45 g 3-Formyl-3-hydroximethyl-tetrahydropyran.

Anschließend wird die überschüssige Menge an Salpetersäure durch Zugabe einer stöchiometrischen Menge Formaldehydlösung zerstört.

Nach Abdestillieren des Wassers wird der Rückstand in wenig Diisopropylketon aufgenommen und durch Abkühlen auskristallisiert. Ausbeute: 58 g (40% bez. auf Aldol) Fp: Schmelzpunkt 140°C unter Decarboxilierung.

b) 41,4 g Tetrahydropyran-3,3-diarbonsäuremethylester werden in 200 ml Xylol gelöst und tropfenweise mit 10,4 g Methylhydrazin versetzt. Dann tropft man 37,2 g 30% Natriummethylatlösung zu. Man steigert die Badtemperatur nun langsam auf 170°C und tropft in dem Maße, wie Methanol abdestilliert, Xylol zu. Bei Raumtemperatur setzt man 50 ml Ether zu und sat ab. Es wird mit Ether gewaschen und getrocknet. 35,8 g Natriumsalz.

12,4 g Natriumsalzes werden mit 70 ml Aceton und 20 mg 15-Krone-5 vorgelegt. Unter Rückfluß tropft man 7,1 g Dimethylcarbaminsäurechlorid zu und hält 4 Stunden am Sieden. Die abgesaugte Acetonphase wird eingeengt und mit Hexan ausgerührt. Ausbeute 4,2 g; Schmelzpunkt 51—53°C.

Elementaranalyse

| | C | H | N |
|---|---|---|---|
| Ber.: | 51,76 | 6,71 | 16,46 |
| Gef.: | 51,7 | 6,7 | 16,5 |

Durch entsprechende Abwandlung der vorstehenden Beispiele wurden die folgenden Verbindungen I hegestellt, deren Schmelzpunkte angegeben sind, oder die als "Öl" bezeichnet sind; die übrigen Verbindungen können unter entsprechender Abwandlung der Herstellvorschriften erhalten werden. Sie lassen aufgrund der Strukturähnlichkeit eine ähnliche Wirkung wie die im einzelnen untersuchten Verbindungen erwarten.

| Nr. | $R^1-O-(CH_2)_n-$ | $R^2$ | $R^3$ | $R^4$ | Fp. (°C)/$n_D^{25}$ |
|---|---|---|---|---|---|
| 3 | $CH_3OCH_2-$ | $C_2H_5$ | $i-C_3H_7$ | $CH_3$ | 56 - 59 |
| 4 | $CH_3OCH_2-$ | $CH_3$ | $CH_3$ | $CH_3$ | 56 - 58 |
| 5 | $CH_3OCH_2-$ | $CH_3$ | $CH_3$ | $OCH_3$ | Öl 1,476 |
| 6 | $CH_3OCH_2-$ | $CH_3$ | $i-C_3H_7$ | $CH_3$ | 41 - 44 |
| 7 | $- CH_2O - CH_2 - CH_2 -$ | | $CH_3$ | $CH_3$ | 51 - 53 |
| 8 | $CH_3OCH_2-$ | $n-C_3H_7$ | $CH_3$ | $CH_3$ | 62 - 65 |
| 9 | $CH_3OCH_2-$ | $n-C_3H_7$ | $C_2H_5$ | $CH_3$ | |
| 10 | $CH_3OCH_2-$ | $i-C_3H_7$ | $CH_3$ | $CH_3$ | 80 - 83 |
| 11 | $CH_3OCH_2-$ | $i-C_3H_7$ | $CH_3$ | $n-C_4H_9$ | |
| 12 | $CH_3OCH_2-$ | $n-C_4H_9$ | $CH_3$ | $CH_3$ | 39 - 42 |
| 13 | $CH_3OCH_2-$ | $n-C_4H_9$ | $cyclo-C_6H_{12}$ | $CH_3$ | |
| 14 | $C_2H_5OCH_2-$ | $n-C_3H_7$ | $CH_3$ | $CH_3$ | |
| 15 | $C_2H_5OCH_2-$ | $n-C_3H_7$ | $i-C_3H_7$ | $CH_3$ | |
| 16 | $C_2H_5OCH_2-$ | $i-C_3H_7$ | $CH_3$ | $CH_3$ | |
| 17 | $C_2H_5OCH_2-$ | $i-C_3H_7$ | $i-C_3H_7$ | $CH_3$ | |
| 18 | $C_2H_5OCH_2-$ | $i-C_3H_7$ | $i-C_3H_7$ | $CH_3$ | |
| 19 | $C_2H_5OCH_2-$ | $n-C_4H_9$ | $CH_3$ | $CH_3$ | |

Die neuen Wirkstoffe interessieren besonders wegen ihrer starken Wirkung gegen Blattläuse. Als Vergleichsmittel wurde deshalb das als Blattlausmittel bekannte Handelsprodukt Pirimicarb gewählt.

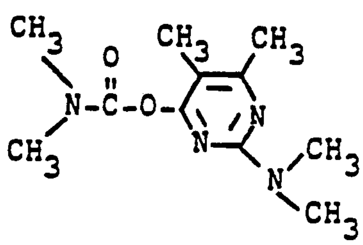

Wirkungsbeispiel A

Dauerkontakt auf Stubenfliegen (Musca domestica)

Petrischalen mit 10 cm Durchmesser werden innen mit der azetonischen Lösung der Wirkstoffe behandelt.

Nach Verdunsten des Lösungsmittels besetzt man die Schalen mit je 20 Stück 4 Tage alten Subenfliegen.

Die Mortalität wird nach 4 Studen ermittelt.

Ergebnis:

| Beanspruchte Verbindung Nr. | mg | % Mort. |
|---|---|---|
| 1 | 0,02 | 100 |
| 3 | 0,01 | 80 |
| 4 | 0,01 | 100 |
| 6 | 0,02 | 100 |
| 7 | 0,2 | 100 |
| 8 | 0,2 | 100 |
| 10 | 0,2 | 100 |

Wirkungsbeispiel B

Kontaktwirkung auf Baumwollwanze (dysdercus intermedius)

Petrischalen mit 10 cm Durchmesser werden mit 1 ml der azetonischen Wirkstofflösung ausgekleidet.

Nach Verdunsten des Lösungsmittels besetzt man die Schalen mit je 20 Larven des vorletzten Stadiums und registriert die Wirkung nach 24 Stunden.

Die Mortalität wird nach 4 Stunden ermittelt.

Ergebnis:

| Beanspruchte Verbindung Nr. | mg | % Mort. |
|---|---|---|
| 1 | 0,01 | 100 |
| 3 | 0,1 | 100 |
| 4 | 0,004 | 100 |
| 6 | 0,02 | 80 |
| 7 | 0,02 | 100 |
| 8 | 0,2 | 100 |
| Vergleichsmittel | 0,2 | <50 |

Wirkungsbeispiel C

Kontaktwirkung auf Blattläuse (Aphis fabae) Spritzversuch

Getopfte Bohnenpflanzen (Vicia faba) mit starkem Blattlausbefall werden in der Spritzkammer mit den wäßrigen Wirkstoffaufbereitungten tropfnaß gespritzt.

Die Auswertung erfolgt nach 24 Stunden.

Ergebnis:

| Beanspruchte Verbindung Nr. | % | % Mort. |
|---|---|---|
| 1 | 0,002 | 100 |
| | 0,001 | ca. 90 |
| 4 | 0,0004 | 100 |
| 7 | 0,001 | 100 |
| Vergleichsmittel | 0,002 | 100 |
| | 0,001 | <60 |

Wirkungsbeispiel D

Systemische Wirkung auf Blattläuse (Aphis fabae); Gießversuch)

Bohnenpflanzen mit starkem Blattlausbefall, die in Plastiktöpfen (ø 8 cm, 300 g Komposterde) stehen, werden mit 20 ml der wäßrigen Aufbereitung der Versuchsmittel angegossen.

Die Feststellung der Mortalitätsrate erfolgt nach 48 Stunden.

Ergebnis:

| Beanspruchte Verbindung Nr. | % | % Mort. |
|---|---|---|
| 1 | 0,001 | ca. 90 |
| 3 | 0,04 | 100 |
| 4 | 0,01 | ca. 90 |
| 6 | 0,02 | 100 |

Wirkungsbeispiel E

Dauerwirkung behandelter Pflanzen auf Blattläuse (Megoura viciae)

Junge Bohnenpflanzen (Vicia faba), die das erste Blattpaar ausgebildet haben, werden in der Spritzkammer mit der wäßrigen Aufbereitung der Wirkstoffe tropfnaß gespritzt. Nach dem Abtrocknen infiziert man die Pflanzen mit adulten Blattläusen. Die Wirkung wird nach 24 Stunden bestimmt.

Tritt eine Mortalitätsrate von 80 bis 100% auf, so infiziert man am 2., 4. bzw. 8. Tag erneut die Pflanzen und beobachtet den Effekt.

Ergebnis:

| Beanspruchte Verbindung Nr. | % | % Mort. |
|---|---|---|
| 1 | 0,01 | 100 |
| 5 | 0,01 | 100 |
| Vergleichsmittel | 0,01 | ca. 80 |

Wirkungsbeispiel F

Megoura viciae, Blattläuse; Dauerwirkung systemischer Mittel

Junge Bohnenflanzen in Plastiktöpfen (ø 8 cm) mit ca. 300 g Erde werden mit 20 ml der wäßrigen Wirkstoffaufbereitung gegossen. Darauf erfolgt die Infektion mit adulten Blattläusen. Nach 48 Stunden kontrolliert man die Wirkung.

Tritt eine Mortalitätsrate über 80% auf, erfolgt eine Neuinfektion. Diese wird nach 4 bzw. 8 Tagen wiederholt.

Ergebnis:

| Beanspruchte Verbindung Nr. | % | nach 8 Tagen % Mort. |
|---|---|---|
| 1 | 0,01 | 100 |
| 5 | 0,01 | ca. 80 |
| Vergleichsmittel | 0,01 | $<60$ |

Wirkungsbeispiel G

Kontaktwirkung auf Zecken (Ornithodorus moubata)

Die Prüfung erfolgt an jungen Zecken, die erst einmal Blut aufgenommen haben. Je 5 Tiere werden in einen Tee-Aufgußbeutel gebracht und 5 Sekunden in die wäßrige Wirkstoffaufbereitung getaucht. Nach 48 Stunden ermittelt man die Mortalitätsrate.

Die Versuchstemperatur beträgt 25 bis 26°C.

Ergebnis:

| Beanspruchte Verbindung Nr. | % | % Mort. |
|---|---|---|
| 1 | 0,04 | 100 |
| 3 | 0,004 | 100 |
| 4 | 0,001 | 80 |
| 5 | 0,1 | 100 |
| 6 | 0,004 | 100 |
| 7 | 0,1 | 100 |
| 8 | 0,04 | 100 |
| Vergleichsmittel | 0,1 | 60 |

**Patentansprüche**

1. Carbaminsäureester der Formel I

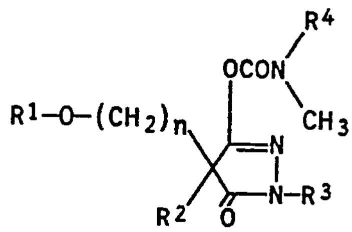

(I),

in der entweder $R^1$ einen verzweigten oder unverzweigten Alkylrest mit bis zu 4 C-Atomen und $R^2$ einen verzweigten oder unverzweigten Alkylrest mit bis zu 6 Kohlenstoffatomen bedeutet, oder $R^1$ und $R^2$ zusammen mit dem Molekülteil, das sie einschließen, einen Tetrahydrofuran- oder Tetrahydropyranring bilden, n die Zahl 1 oder 2 bedeutet, $R^3$ einen verzweigten oder unverzweigten Alkylrest mit bis zu 6 C-Atomen oder einen Cycloalkylrest mit 3—6 C-Atomen und $R^4$ den Methyl- oder Methoxyrest bedeuten.

2. Carbaminsäureester der Formel I gemäß Anspruch 1, dadurch gekennzeichnet, daß $R^3$ und $R^4$ Methyl bedeuten.

3. Insektizides und akarizides Mittel, enthaltend einen Carbaminsäureester der Formel I gemäß Anspruch 1, gegebenenfalls mit inerten Zusatzstoffen.

4. Verfahren zur Bekämpfung von Schädlingen, dadurch gekennzeichnet, daß man eine wirksame Menge eines Carbaminsäureesters der Formel I, gemäß Anspruch 1 auf die Schädlinge und/oder deren Lebensraum einwirken läßt.

5. Verfahren zur Herstellung eines insektiziden und/oder akariziden Mittels, dadurch gekennzeichnet, daß man jeweils mindestens einen Carbaminsäureester der Formel I gemäß Anspruch 1 mit einer oberflächenaktiven Verbindung und/oder einem Streckmittel und/oder einem Wirkstoff gleicher Wirkungsrichtung mischt.

6. Verfahren zur Herstellung von Carbaminsäureestern der Formel I gemäß Anspruch 1, dadurch gekennzeichnet, daß man das Salz eines entsprechenden Pyrazolons der Formel II,

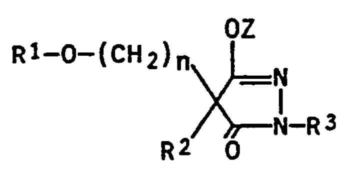

II

worin Z ein Aquivalent eines ein- oder mehrwertigen Kations bedeutet, mit einem entsprechenden Carbaminsäurehalogenid der Formel III

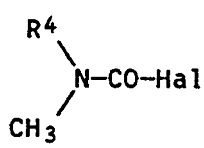

III

umsetzt.

7. Verfahren zur Herstellung von Carbaminsäureestern der Formel I, gemäß Anspruch 1, dadurch gekennzeichnet, daß man ausgehend von einem entsprechenden 2-Alkylacrolein $H_2C=CR^2CHO$, Formaldehyd und einem entsprechenden Alkanol $R^1OH$ ein Aldol

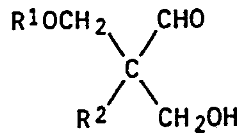

herstellt, dieses mit Salpetersäure oxidiert, die gebildete Malonsäure verestert, den Ester mit einem entsprechenden Hydrazin $H_2N—NHR^3$ zum Pyrazolinon umsetzt und dieses carbamoyliert.

**Revendications**

1. Esters d'acide cabamique de formule I

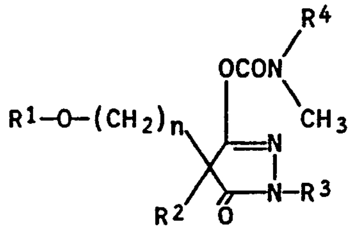

(I),

dans laquelle $R^1$ représente un reste alkyle ayant jusqu'à 4 atomes C, ramifié ou non ramifié et $R^2$, un reste alkyle ayant jusqu'à 6 atomes de carbone, ramifié ou non ramifié, ou $R^1$ et $R^2$ représentent ensemble, avec la partie de molécule incluse, un noyau tétrahydrofuranne ou tétrahydropyranne, *n* représente le nombre 1 ou 2, $R^3$ un reste alkyle ayant jusqu'à 6 atomes C, ramifié ou non ramifié, ou un reste cycloalkyle à 3 à 6 atomes C, et $R^4$ le reste méthyle ou méthoxy.

2. Esters d'acide carbamique de formule I selon la revendication 1, caractérisé par le fait que $R^3$ et $E^4$ représentent méthyle.

3. Agent insecticide et acaricide, contenant un ester d'acide carbamique de formule I selon la revendication 1, éventuellement avec addition d'adjuvants inertes.

4. Procédé pour la lutte contre des parasites, caractérisé par le fait que l'on fait agir sur les parasites et/ou leur isotope une quantité efficace d'un ester d'acide carbamique des formule I selon la revendication 1.

5. Procédé de préparation d'un agent insecticide et/ou acaricide, caractérisé par le fait que l'on mélange dans chaque cas au moins un ester d'acide carbamique de formule I selon la revendication 1 avec un composé tensio-actif et/ou une charge et/ou un principe actif d'une même orientation d'action.

6. Procédé de préparation d'esters d'acide carbamique de formule I selon la revendication 1, caractérisé par le fait que l'on fait réagir le sel d'une pyrazolone appropriée, de formule II

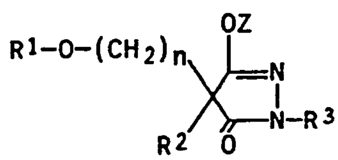

II

dans laquelle Z représente l'équivalent d'un cation mono- ou polyvalent, avec un halogénure d'acide carbamique approprié, de formule III

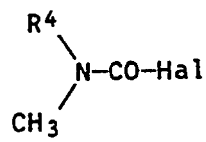

III

7. Procédé de préparation d'esters d'acide carbamique de formule I selon la revendication 1, caractérisé par le fait que partant d'une 2-alkylacroléine $H_2C=CR^2CHO$ appropriée, de formaldéhyde et d'un alcanol $R^1OH$ approprié, on prépare un aldol

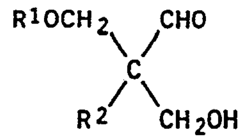

on oxyde celui-ci avec de l'acide nitrique, on estérifie l'acide malonique formé, on met à réagir l'ester avec une hydrazine $H_2N—NHR^3$ appropriée pour obtenir la pyrazolinone et on carbamoyle cette dernière.

## Claims

1. A carbamate of the formula I

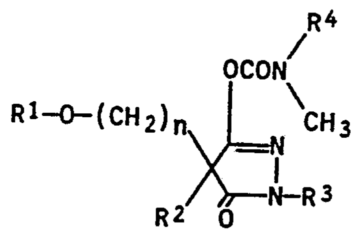

(I),

where either $R^1$ is straight-chain or branched alkyl of not more than 4 carbon atoms and $R^2$ is straight-chain or branched alkyl of not more than 6 carbon atoms, or $R^1$ and $R^2$, together with the molecular moiety which they include, form a tetrahydrofuran or tetrahydropyran ring, n is 1 or 2, $R^3$ is straight-chain or branched alkyl of not more than 6 carbon atoms or cycloalkyl of 3 to 6 carbon atoms and $R^4$ is methyl or methoxy.

2. A carbamate of the formula I as claimed in claim 1, where $R^3$ and $R^4$ are each methyl.

3. An insecticidal and acaricidal agent containing a carbamate of the formula I as claimed in claim 1 and with or without inert additives.

4. A process for controlling pests, wherein an effective amount of a carbamate of the formula I as claimed in claim 1 is allowed to act on the pests and/or their habitat.

5. A process for the preparation of an insecticidal and/or acaricidal agent, wherein in each case at least one carbamate of the formula I as claimed in claim 1 is mixed with a surfactant and/or an extender and/or an active ingredient having the same direction of action.

6. A process for the preparation of a carbamate of the formula I as claimed in claim 1, wherein the salt of a corresponding pyrazolone of the formula II

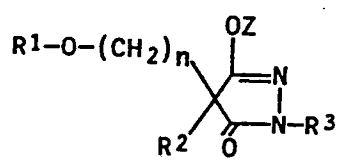

II

where Z is one equivalent of a monovalent or polyvalent cation, is reacted with a corresponding carbamyl halide of the formula III

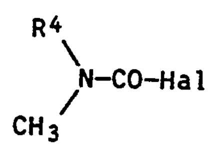

III

7. A process for the preparation of a carbamate of the formula I as claimed in claim 1, wherein an aldol

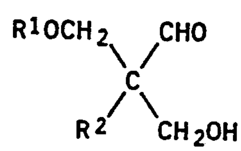

is prepared from a corresponding 2-alkylacrolein $H_2C=CR^2CHO$, formaldehyde and a corresponding alkanol $R^1OH$, this aldol is oxidized with nitric acid, the malonic acid formed is esterified, the ester is reacted with a corresponding hydrazine $H_2N—NHR^3$ to give the pyrazolinone, which is then reacted to give the carbamate.